# EUROPEAN PATENT SPECIFICATION

(11) **EP 2 140 880 B1**
(45) Date of publication and mention of the grant of the patent: **14.11.2012**
(21) Application number: 08159687.6
(22) Date of filing: 04.07.2008
(51) Int. Cl.: A61K 39/35, A61K 39/36, A61P 37/08

(54) **Modification of allergens**
Modifikation von Allergenen
Modification d'allergènes

(43) Date of publication of application: 06.01.2010
(73) Proprietor: HAL Allergy Holding B.V., 2333 CH Leiden (NL)
(72) Inventor: Koppelman, Stefan Johan, 3731 EG De Bilt (NL); van den Hout, Robertus Henricus Joannes Alfonsus, 2023 VD Haarlem (NL); Sleijster-Selis, Henriëtte Emilie, 1901 JE Castricum (NL); Luijkx, Dionisius Marinus Antonius Maria, 6708 JJ Wageningen (NL)
(74) Representative: van Kooij, Adriaan

(56) References cited:
- EP-A- 1 547 610
- US-A- 3 794 630
- US-A- 4 180 562
- CLARE D A ET AL: "Transglutaminase polymerization of peanut proteins." JOURNAL OF AGRICULTURAL AND FOOD CHEMISTRY 24 JAN 2007, vol. 55, no. 2, 24 January 2007 (2007-01-24), pages 432-438, XP002505516 ISSN: 0021-8561
- AKDIS C A ET AL: "REGULATION OF SPECIFIC IMMUNE RESPONSES BY CHEMICAL AND STRUCTURAL MODIFICATIONS OF ALLERGENS" INTERNATIONAL ARCHIVES OF ALLERGY AND IMMUNOLOGY, XX, XX, vol. 121, no. 4, 1 January 2000 (2000-01-01), pages 261-269, XP001016273 ISSN: 1018-2438

## Description

The invention relates to a process for modifying allergens to enhance their suitability in immunotherapy. The invention further relates to the modified allergens and pharmaceutical compositions thereof, as well as to their use in immunotherapy.

Allergens are substances that can cause an allergic reaction or induce an allergy. In people who have an allergy, allergens are recognized by the immune system as "foreign" or "dangerous", whereas they cause no response in most other people. Examples of common allergens are, or are present in, bacteria, viruses, animal parasites, insect venoms, house mite, chemicals, dust, drugs such as antibiotics, foods, perfumes, plants, pollen, and smoke. Food allergy is mainly associated with a limited range of foodstuffs such as peanuts, tree nuts, hen's eggs, cow's milk, wheat (gluten), soybeans, fish and shellfish. The prevalence of food allergy is approximately 1-2% in adults and 6-8% in children.

The occurrence of allergic reactions is associated with a reaction of an individual's immune system to exposure of a particular allergen. In an individual having the tendency to develop an allergy to a particular allergen, first time exposure normally does not give rise to any allergic reactions. The allergen is taken up by an antigen presenting cell (APC), such as a macrophage or dendritic cell, which degrades the allergen. Fragments of the allergen are presented to CD4+ T-cells, which may respond in essentially two different ways. T-cells secrete cytokines which have effects on other cells of the immune system, most notably B-cells. They are subdivided into two categories. The first category contains Thelper1-cells, secreting a.o. interleukin-2 (IL-2) and interferon-γ (IFN-γ). The presence of IFN-γ will induce B-cells to produce specific subclasses of IgG antibodies. The second category contains Thelper2-cells. These secrete different cytokines such as IL-4, IL-5 and IL-13. Production of IL-4 and IL-13 are necessary for the initiation and maintenance of IgE antibodies produced by B cells. These IgE antibodies will have specificity for the allergen.

Upon every additional exposure of the individual to the particular allergen, said allergen will bind to the available IgE antibodies, particularly to those bound to the surface of mast cells or basophils. As allergens typically have several sites that can bind to the IgE antibodies, those antibodies in effect become crosslinked. The result of the crosslinking of the surface-bound IgE antibodies is that the mast cells and basophils degranulate and release mediators like histamines that trigger allergic reactions.

Treatment of allergies is difficult. Many allergic individuals try to adapt to their situation and avoid exposure to the substances to which they are allergic. The feasibility of such behaviour adaptation will depend of course to a great extent on the type of allergy. For instance, it is easier to avoid intake of certain foodstuffs than it is to avoid exposure to pollen. Therapies involving drugs, such as antihistamines, decongestants, or steroids, are available but only combat the symptoms of an allergic reaction. They do not prevent that future exposure to the allergen causes new allergenic reactions.

In the past, it has been proposed to treat allergies on the basis of immunotherapy. Such treatment involves repeated injections of allergen extracts over a long period to desensitize a patient to the allergen. This therapy is, however, very time consuming, usually involving years of treatment, and frequently fails to achieve the goal of desensitizing the patient to the allergen. Moreover, particularly for food allergies or allergies to insect venoms, it is not a safe treatment. With many food and insect venom allergies, allergic reactions are associated with a significant risk of anaphylaxis, which is a systemic and potentially lethal type of allergic reaction. In clinical trials for immunotherapy for peanut allergy, anaphylactic events occurred, once with fatal outcome (see Oppenheimer et al., J. Allergy Clin. Immunol., 1992 Aug., 90(2), pp. 256-262). In order to reduce the anaphylactic adverse events observed during insect venom immunotherapy, pre-treatment with antihistamines is recommended, indicating the poor safety profile of current immunotherapy for insect venoms (see Scribner et al., Curr. Opin. Allergy Clin. Immunol., 2003, 4, pp. 295-298).

It has been proposed to modify allergens to reduce the risk of such dangerous side effects. The modification aims to reduce the allergenic reactions caused by the allergen, while retaining its immunogenicity. Thus, ideally exposure to the modified allergen by an allergy patient would elicit the desired immune response so that, in time, the patient is desensitized to the allergen without causing severe allergic reactions during the therapy.

A known modification of allergens is a treatment with glutaraldehyde, which causes cross-linking of allergen proteins. The aldehyde groups of this glutaraldehyde react with the amino groups of lysine residues in the protein, or with the N-terminus. When the two aldehyde groups of glutaraldehyde react with amino groups of different proteins, a cross-link is made. For allergens, such cross-linking may lead to cross-linked material of variable size, with altered immunological characteristics (Patterson et al., J. Allergy Clin. Immunol., January 1979, 63(1):47-50). It has been demonstrated for some allergens, like from tree pollen or grass pollen, that modification with glutaraldehyde leads to diminished IgE-binding, which reduces side effects in immunotherapy. The explanation for this is that lysine residues in allergens may be involved in IgE-epitopes, and that modification or cross-linking of these lysine residues leads to diminished IgE binding due to alterations in the conformation of the protein structure.

However, recently the ability of glutaraldehyde-treated allergens to stimulate T-cells has been disputed (see Würtzen et al., Int. Arch. Allergy Immunol., 2007, 144(4):287-95). Furthermore, it has been found that treatment with glutaraldehyde is not always suitable. In particular, the IgE binding of various allergens, such as peanut allergens, modified by treatment with glutaraldehyde is not reduced which means that allergic reactions still occur.

In WO 2005/060994 it has been disclosed that food allergens, in particular seed storage proteins such as 2S albumins, may be modified by reduction and alkylation. It is postulated that this treatment results in both breakage of disulfide bonds and prevention of reformation of disulfide bonds. The modification is stated to result in a reduction or even prevention of the production specific IgE antibodies after presenting an individual's immune system with the modified allergen. However, IgE-binding itself was not investigated. Furthermore, it has been found that the allergenicity of the allergens in some cases needs to be reduced even further, without reducing immunogenicity, for them to be suitable and safe in an effective immunotherapy. For instance, some allergens, such as wasp or bee venom, tend to provoke notoriously severe allergic reactions in people who are allergic to them so that immunotherapy in cases of this type of allergies has hitherto not been sufficiently safe. Furthermore, the allergens modified in accordance with this prior art docu ment are not always sufficiently stable as reduction of disulfide bridges of highly structured proteins leads to increased susceptibility for proteolysis and heat denaturation.
Akdis (Int. Arch Allergy Immunol 2000; 121:261-269) discloses differential regulation of allergen-specific T cell cytokine patterns and IgE:IgG production was demonstrated by modification by reduction and alkylation of the three-dimensional structure of allergens because of linearity in T cell epitopes and conformation dependence in B cell epitopes.

The present invention provides an improved way of modifying allergens which greatly reduces the allergenicity of allergens, essentially without detrimentally affecting their immunogenicity. A modification according to the invention can be used for a great variety of allergens. Because the allergens modified according to the invention display a significantly improved safety profile compared to currently available allergen products, the invention provides a means to improve existing immunotherapies. In particular, allergy patients will experience no or less severe adverse effects of the immunotherapy when using allergens modified according to the invention. Also, the efficacy of the treatment may be improved as it will be possible to treat patients with higher doses of the allergens which, in turn, may decrease the time for the patient to become tolerant. In addition, the invention provides a means to develop immunotherapy for allergies that are caused by allergens which are unsuitable for immunotherapy since their allergenicity cannot be sufficiently modified with currently available methods.

A modification according to the invention comprises the steps of reduction and treatment with a cross-linking agent, such as glutaraldehyde and further alkylation.

Reduction is carried out prior to alkylation and treatment with the cross-linking agent is carried out after reduction and alkylation.

Surprisingly, exposure of an allergic individual to an allergen modified according to the invention is not only safe and does not cause any significant allergic reactions, it is also possible to effectively desensitize the individual to the allergen. Presenting the individual's immune system with an allergen modified in accordance with the invention has been found to lead to a reduction or prevention of the production of specific-IgE antibodies. In an individual with a developed allergy, the IgE response of the immune system may be down-regulated skewing the immune response from a Thelper-2 mediated reaction towards a Thelper-1 mediated reaction. As a result, after completion of the treatment, the individual no longer needs to avoid exposure to the allergens to which he is allergic, e.g. intake of foodstuffs containing the dietary protein to which he used to be allergic no longer needs to be avoided.

It is further advantageous that an allergen that is modified in accordance with the invention is highly stable and very safe. Immunotherapy for allergies to highly dangerous allergens, such as, but not limited to, peanut or wasp or bee venom, has been made possible with allergens modified according to the invention.

The term "allergen" is used herein to refer to a substance which, when exposed to a mammal, will be able to mount an immune response resulting in antibodies of the IgE-class and which also will be able to trigger an allergic reaction when the mammal later on is exposed again to the substance. Allergens in terms of the present invention are allergenic proteins, which may consist of protein or a protein combined with a lipid or carbohydrate such as a glycoprotein, a proteoglucan, a lipoprotein etc. An extensive overview of known allergens and their nomenclature is published by the International Union of Immunological Societies (see www.allergen.org).

In accordance with the invention, the allergen typically is a protein, comprising cystein residues. More preferably, the allergen comprises cystein residues that form disulfide bridges or disulfide bonds, preferably intramolecular disulfide bonds. In the context of the invention, the terms "disulfide bridges" and "disulfide bonds" will be used interchangeably. It is further preferred that the allergen is from a vegetable source, preferably a storage protein, from an insect, a mammal or a fish or crustacean, or from an expression system for recombinant proteins like a bacterium, yeast or other microorganism.

Allergens from plants may be subdivided in allergens from pollen and the like and allergens from seeds. Allergens from seeds are preferably storage proteins such as 2S-albumin or conglutin. In purified form such storage proteins are, in a preferred embodiment, for instance Ara h 2 or Ara h 6 from peanut. Alternatively, allergens from plants may be subdivided in allergens from fruit, such as lipid transfer proteins, allergens from oil crops, such as peanut or soybean, and allergens from treenuts and seeds such as hazelnut, walnut and sunflower seed. Allergens from insects are preferably venoms from for instance bee or wasp, which may be purified to obtain individual allergens.

Prior to modification, the allergen is preferably isolated (purified) from its biological source, such as (a part of) the animal, insect venom, foodstuff, or the like. It is, however, also possible to modify a crude, or partially purified extract comprising the allergen together with other components of the biological source. Although this may result in administration to a patient of other proteins or other substances modified by reduction and treatment with a cross-linking agent, this is not considered to be harmful. Therefore, the present invention pertains to modification of isolated allergens as well as to crude extracts from allergen-containing products, such as food items, as obtainable by e.g. milling, grinding, etc. which have been subjected to modification according to the present invention. It is also possible to use mixtures of allergens, particularly mixtures of allergens from one source.

If desired, isolation of the allergen may be done by any known method, such as methods involving extraction and liquid chromatography. Methods for isolating allergens from various biological sources are known per se and may be conveniently adapted to the needs of the circumstances by the skilled person based on his common general knowledge.

The allergen may also be obtained commercially, such as for instance from Greer, Lenoir, NC, USA, from Indoor Biotech, Charlottesville, NC, USA, from Allergon AB, Angelholm, Sweden, from ALK Albello, Horsam, Denmark, or from Pharmacia Diagnostics AB, Uppsala, Sweden. It is further possible to use allergens that have been obtained by recombinant means or to use synthetic peptides as allergen. Recombinant allergens are commercially available from for instance BioMay, Vienna, Austria. Synthetic peptides that can be used as allergens are commercially available from for instance Circassia, Oxford, UK.

In accordance with the invention, the allergen is modified by reduction and treatment with a cross-linking agent and further alkylation. Treatment with the cross-linking agent is carried out after reduction and alkylation. Reduction is carried out prior to alkylation. Sulfite can be used to modify disulfide bridges into SO₃- groups, thereby preventing re-oxidation in a similar way as 4,5-dihydroxy-1,2-dithiane and 2-({4-[(carbamoylmethyl)-sulfanyl]-2,3-dihydroxybutyl}sulfanyl)acetamide do (see below). In general, reductive alkylation in a single step as described by Means and Feeney may be applied to reduce disulfide bridges irreversibly in a single step.

Without wishing to be bound by theory, it is believed that IgE binding of allergens, that cause allergic reactions, is reduced quicker as a result of a modification according to the invention than the T-cell activation, that causes the desired immune response in immunotherapy. It is hypothesized that the IgE binding is more dependent on the conformational structure of the allergen, whereas the T-cell activation is believed to be more dependent on the linear structure of the allergen (see also Larche, J. Allergy Clin. Immunol. 2007,119(4), pp. 906-909). The optimal degree of modification according to the invention, which is a balance of reduction, alkylation and treatment with a cross-linking agent, will differ for every allergen, but may be found readily by a person of ordinary skill in the art based on the considerations presented herein.

These considerations include the goal to achieve maximal reduction, and alkylation, and only limited modification with the cross-linking agent, in order to achieve a reduction in IgE-binding while retaining T-cell activation as much as possible. If cross-linking is carried to a too great extent, this may lead to an undesired modification of the T-cell epitope and thus to a reduction in T-cell activation. Reference is made in this regard also to the solid-phase IgE-binding assays as illustrated in the Examples.

Reduction and alkylation of proteins are protein modifications that are known per se. For an overview, reference is made to Herbert et al., Electrophoresis (2001), 22:2046. It will be understood that it is preferred that only reagents are used which lead to modified allergens that are acceptable in the context of the production of foodstuffs or pharmaceuticals.

In a preferred embodiment, reduction is performed using a reducing agent chosen from the group of 2-mercaptoethanol (β-ME), dithiothreitol (DTT), dithioerythritol, cysteine, homocystein, tributylphosphine, sulfite, tris(2-carboxyethyl) phosphine (TCEP), sodium (cyano) borohydride, lye, glutathione, E-mercapto ethylamine, thioglycollic acid, methyl sulfide, ethyl sulfide and combinations thereof. In general, alkylthiol compounds (R-SH) provide suitable reducing agents. Preferably, those reducing agents are used that disrupt the disulfide bonds while maintaining other chemical characteristics of the protein. For instance, NH₂ groups are preferably left intact.

Alternatively, reduction may be performed by using enzymatic means, such as by using proteins that catalyse thiol-disulfide exchange reactions such as for instance glutaredoxin or thioredoxin. Such proteins may exert their effect via two vicinal (CXYC) cysteine residues, which either form a disulfide (oxidized form) or a dithiol (reduced form). Alternatively proteins may be used that are capable of catalysing the rearrangement of both intrachain and interchain-S-S-bonds in proteins such as protein disulfide isomerase or other polypeptides capable of reducing disulfide bonds such as for instance disclosed in WO 00/70064.

Preferably, the reduction reaction is continued until the reaction stops and essentially all disulfide bonds in the allergen have been broken. The conditions under which reduction is carried out can be optimised depending on the chosen reducing agent by the skilled person based on his general knowledge. Typically, reduction will be carried out at neutral, or near neutral pH, preferably at a pH between 6 and 8, at concentrations of reducing agents in the suitable range of, or equivalent to, for instance about 1-100 mM of DTT (or **β-ME),** possibly by using a suitable buffer. An example of a suitable buffer comprises chaotropic reagents, such as guanidine and/or urea, which may result in (reversible) unfolding of the allergen protein. If such reagents are used, it is preferred that reduction and alkylation are performed before treatment with the cross-linking agent. The temperature during reduction will generally lie between ambient or room temperature and 100°C, optionally under a reducing atmosphere, such as an anoxic atmosphere, preferably a nitrogen (N₂) atmosphere. Of course, care should be taken that the allergen does not denature during **the** reaction.

Alkylation is preferably carried out by blocking the SH-radicals that result from the cleavage of the disulfide bonds during reduction. Preferred alkylation reagents are chosen from the group of N-ethylmaleimide, cystamine, iodoacetamide, iodoacetic acid. More generally, at least one disulfide bond can be reduced and alkylated to produce cysteine residues with side chains having the chemical formula -CH₂-S-[CH₂]ₙ-R' wherein n is an integer between 1 and 5 and R' is selected from the 1-5 carbon groups consisting of alkyl groups (e.g., methyl, ethyl, n-propyl, etc.); carboxyalkyl groups (e.g., carboxymethyl, carboxyethyl, etc.); cyano alkyl groups (e.g., cyanomethyl, cyanoethyl, etc.); alkoxycarbonyl alkyl groups (e.g., ethoxycarbonylmethyl, ethoxycarbonylethyl, etc.); carbomoylalkyl groups (e.g., carbamoylmethyl, etc.); and alkylamine groups (e.g., methylamine, ethylamine, etc.). Other suitable alkylating reagents include alkylhalogenides; alkylsulfates; alkenes, preferably terminal alkenes (H₂C)=C(H)-R; and other alkylating reagents known to one skilled in the art. Alternatively, alkylation may be performed by using enzymatic means, such as by using sulfhydryl oxidase, for instance as may be obtained from chicken egg protein. Although strictly not an alkylation reaction, the oxidation of the SH-radicals towards for instance SO₂ or SO₃ forms an aspect of the present invention since the reformation of the protein disulfide bonds is effectively blocked as a result thereof.

In some preferred embodiments of the invention, the alkylation will introduce amino groups that may react with the cross-linking agent in embodiments where this step is performed after alkylation. This may be used as a further instrument to achieve a desired degree of modification of the allergen. Examples of suitable alkylation reagents in accordance with this embodiment are cystamine, iodoacetamide, acrylamide, and 2-({4-[(carbamoylmethyl)sulfanyl]-2,3-dihydroxybutyl}sulfanyl)acetamide.

Typically, alkylation will be carried out at neutral, or near neutral pH, preferably at a pH between 6 and 8, possibly be using a suitable buffer. An example of a suitable buffer comprises chaotropic reagents, such as guanidine and/or urea, that may result in unfolding of the allergen protein. If such reagents are used, it is preferred that reduction and alkylation are performed before treatment with the cross-linking agent. The temperature during alkylation will generally lie between ambient or room temperature and 50°C.

The allergen is in accordance with the invention also treated with a cross-linking reagent. The cross-linking agent may be a bifunctional reagent, which may be a homo-bifunctional reagent or a hetero-bifunctional reagent. This means that it may comprise either two of the same functional moieties or that it may comprise two different functional moieties. By virtue of its bifunctionality, the bifunctional reagent may act as a cross-linking agent. However, other cross-linking agents, such as certain monoaldehydes, may also be used. The functional moieties of the cross-linking agent may react with certain amino acids in the allergen protein. For instance, aldehyde groups of a cross-linking moiety may react with the amino groups of lysine residues In the protein, or of the N-terminus. In the case of formaldehyde, for instance, the product of this reaction is very reactive as a result of which both inter- and intramolecular cross-links may be formed.

Suitable examples of cross-linking agents are aldehydes, such as formaldehyde and glutaraldehyde. An overview of suitable cross-linking agents is provided by Means and Feeney, Anal. Biochem., 1995, 224, pp. 1-16, J. Food Biochem., 1998, 22, pp. 399-425, and Bioconjugate Chem., 1990, 1, pp. 2-12. Preferably, the cross-linking agent is glutaraldehyde. This treatment may be performed at conditions that can be easily optimized by the skilled person based on his common general knowledge and the considerations set forth by Means and Feeney. They may comprise reacting the allergen with the crosslinking agent in a molar ratio of 10-100 : 1 of cross-linking agent to lysine residues, at highly alkaline pH, at room temperature for a few hours. The reaction may be stopped in any suitable way, for instance by addition of an excess of glycine followed by diafiltration. By choosing the optimal process parameters, such as reaction time, temperature, concentration of the allergen and of the cross-linking agent, and so on, the size, the secondary structure and the tertiary structure of the modified allergen that is obtained can be influenced, thereby fine-tuning its immunological properties, such that the IgE-binding is strongly diminished while maintaining T-cell reactivity.

The invention also encompasses a modified allergen that can be obtained by the above described modification reactions.

The above-described modified allergen is, in accordance with the invention, used in immunotherapy. In this context, it may be administered in any suitable dosage form.

The modified allergen may be used in treatment of an allergy brought about by the native form of the allergenic protein from which the modified allergen is derived. It may also be used in treatment of an allergy that is cross-reactive with the allergenic protein. There are many examples of cross-reactive allergies, where an allergic reaction is provoked by exposure to an allergen from a different source than the substance to which the subject is allergic. For instance, birch pollen allergens are known to be used in immunotherapy of various food allergies, such as apple allergy. Also, an allergen derived from one particular grass species may be used to treat allergies for many grass species.

It will be understood that the invention also relates to a pharmaceutical composition comprising the modified allergen for immunotherapy directed against allergy. A pharmaceutical composition of the invention comprises a therapeutically effective amount of the modified polypeptides of the claimed invention. Once formulated, the pharmaceutical compositions of the invention can be administered directly to the subject. Direct delivery of the compositions will generally be accomplished by injection, but the compositions may also be administered orally, nasally, rectally, mucosally, through the skin, subcutaneously, sublingually, intraperitoneally, intravenously, intralymphatically or intramuscularly, pulmonarily, or delivered to the interstitial space of a tissue.

The pharmaceutical composition may also comprise a suitable pharmaceutically acceptable carrier and may be in the form of a capsule, tablet, lozenge, dragee, pill, droplets, suppository, powder, spray, vaccine, ointment, paste, cream, inhalant, patch, aerosol, and the like. As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the modified allergen. It may be preferred to further include an adjuvant, preferably one known to skew the immune response towards a Thelper-1 mediated response, in the dosage form, in order to further stimulate or invoke a reaction of the patient's immune system upon administration. Suitable adjuvants include such adjuvants as complete and incomplete Freund's adjuvant and aluminium hydroxide, the latter of which works through a depot effect. It is also conceived that the modified allergen is incorporated in a foodstuff and is administered to a patient together with food intake.

A pharmaceutical composition may also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral add salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

For therapeutic treatment, modified allergenic proteins may be produced as described above and applied to the subject in need thereof. The modified allergenic proteins may be administered to a subject by any suitable route, preferably in the form of a pharmaceutical composition adapted to such a route and in a dosage which is effective for the intended treatment. Therapeutically effective dosages of the modified allergenic proteins required for decreasing the allergenic reaction to the native form of the protein or for desensitising the subject can easily be determined by the skilled person, e.g. based on the clinical guidelines for immunotherapy for allergy treatment. In particular, this is practiced for immunotherapy for insect venoms.

The term "therapeutically effective amount" as used herein refers to an amount of a therapeutic, *viz*. a modified allergenic protein according to the present invention, to reduce or prevent allergic reactions to allergenic proteins, or to exhibit a detectable therapeutic or preventative effect. The effect can be detected by, for example, reduced IgE levels to the allergenic protein and increased thresholds for challenge by allergens. The precise effective amount for a subject will depend upon the subject's size and health, the nature and extent of the condition, and the therapeutics or combination of therapeutics selected for administration. In case a subject has undergone treatment with antihistamines, dosages will typically tend to be higher than without such pre-treatment. Thus, it is not useful to specify an exact effective amount in advance. However, the effective amount for a given situation can be determined by routine experimentation and is within the routine judgment of the clinician or experimenter. Specifically, the compositions of the present invention can be used to reduce or prevent allergic reactions to allergenic proteins and/or accompanying biological or physical manifestations. Such manifestations may include contraction of smooth muscle in the airways or the intestines, the dilation of small blood vessels and the increase in their permeability to water and plasma proteins, the secretion of thick sticky mucus, and, in the skin, redness, swelling and the stimulation of nerve endings that results in itching or pain. Manifestations that may be prevented by immunotherapy according to the present invention include skin manifestations such as rashes, hives or eczema; gastrointestinal manifestations including cramping, nausea, vomiting or diarrhoea; or respiratory manifestations including sneezing or runny nose, coughing, wheezing or shortness of breath. Other manifestations that may be prevented include itching of skin, flushes, congestion, eye irritation, asthma, itching in the mouth or throat which may progress to swelling and anaphylaxis. Methods that permit the clinician to establish initial immunotherapy dosages are known in the art (e.g. US patent 4,243,651). The dosages to be administered must be safe and efficacious. As with any medical treatment, a balance must be struck between efficacy and toxicity.

For purposes of the present invention, an effective dose will be from about 0.1 ng/kg to 0.1 mg/kg, 10 ng/kg to about 10 µg/kg, or 0.1 µg/kg to 1 µg/kg of the modified allergenic protein relative to the body weight of the individual to which it is administered. Often, a treatment will comprise starting with the administration of dosages at the lower end of these ranges and increasing the dosages as the treatment progresses. These dosages are intended for modified allergens obtained from purified allergens. For modified allergens based on a crude extract of the allergen, dosages may be higher corresponding to the purity of the extract used.

For typical desensitization treatment, it is typically necessary for the patient to receive frequent administrations, e.g., initially every two or three days, gradually reducing to once every two or three weeks. Other suitable desensitisation programs include subcutaneous injections once every 2-4 weeks the dosage of which injections may gradually increase over a period of 3-6 months, and then continuing every 2-4 weeks for a period of up to about 5 years. It is also possible, particular for sublingual administration, that daily administrations are given.

Desensitization protocols may also comprise a form of treatment conventionally known in various equivalent alternative forms as rapid desensitization, rapid allergen immunotherapy, rapid allergen vaccination, and rapid or rush immunotherapy. In broad terms, this procedure aims to advance an allergic patient to an immunizing or maintenance dose of extract (i.e., allergen) by administering a series of injections (or via another suitable carrier) of increasing doses of the allergen at frequent (e.g. hourly) intervals. If successful, the patient will exhibit an improved resistance to the allergen, possibly even presenting a total non-reactivity to any subsequent allergen exposure. Various desensitization protocols are known in the art and may for instance comprise a method of treating a patient having an immediate hypersensitivity to an allergen using an accelerated rapid immunotherapy schedule in combination with a method of pre-treating such patient with prednisone and histamine antagonists prior to receiving the accelerated immunotherapy such as described in US patent application 2003/082212.

Yet in another alternative embodiment, the modified allergens or compositions of the invention may be administered from a controlled or sustained release matrix inserted in the body of the subject.

It will be understood, that the optimal dose and administration scheme of how to reach this dose may vary per patient. Based on his common general knowledge and taking due account of IgE-mediated side effects, the skilled person will be able to optimize dosage and administration scheme. By way of example, reference is made to a treatment of allergic rhinitis described by Bousquet et al., Allergis Rhinitis and its Impact on Asthma (ARIA) 2008 update in collaboration with the World Health Organization, GA(2)LEN and AllerGen, Allergy, April 2008, 63 Suppl. 86:8-160, and a venom immunotherapy described by Bilò et al., Curr. Opin. Allergy Clin. Immunol., December 2007, 7(6):567-73. For example, in a 3 months period the allergen may be given weekly, with weekly increasing doses until a maintenance dose, e.g. 100 micrograms, is reached. However, if treatment with this maintenance dose does not result in sufficient protection, the dose may be increased. An advantage of an allergen modified according to the invention is that it binds to IgE to a lower extent. This may prevent IgE-mediated side effects and allow quicker up-dosing.

The invention will now be elucidated by the following, non-restrictive examples.

### EXAMPLES

### MATERIALS AND METHODS

### Peanut protein extraction and purification

Peanut extract was prepared using commercially available peanut meal. Purified Ara h1 and Ara h 2 are available at TNO (Zeist, The Netherlands) and described in detail by Koppelman et al., Clin. Exp. Allergy, April 2005, 35(4):490-7. In short, lyophilized crude peanut extract (CPE) was dissolved in 20 mM TRIS-bis-propane, pH 7.2 (TBP) to a final concentration of 1 mg/mL. Undissolved particles were removed by centrifugation (3000 xg, 15 min) and the solution was applied on a 8 mL Source Q column (1 × 10 cm) previously equilibrated with TBP. After washing the column with 80 mL of TBP, a linear gradient of 200 mL (0-1 M NaCl in TBP) was applied to elute the bound proteins (2 mL/min). To remove traces of peanut lectin from Ara h2 (less than1%), Ara h2 was dialysed against 50 mM NaAc, pH5.0 and loaded on a 1 mL Source S column (0.5 × 5 cm) equilibrated with 50 mM NaAc, pH 5.0. After washing with 10 mL of 50 mM NaAc, pH 5.0 the column was eluted using a 25 mL linear gradient (0-500 mM NaCl in 50 mM NaAc, pH 5.0) with a flow velocity of 0.25 mL/min.

Ara h6 was purified according to earlier described procedures (Koppelman et al., Clin. Exp. Allergy, April 2005, 35(4):490-7), ammonium sulphate was added to the crude extract to attain a concentration of 40% saturation at 4°C. The solution was centrifuged (45 min, 8000 xg, at 4°C). The cold supernatant was filtered over glass wool to remove fat particles. Ammonium sulphate was then added to a concentration of 80% saturation at 4°C. The solution was centrifuged again (45 min, 10 000 xg, at 4°C). The pellet was then resuspended in 1.3 L 20 mm Tris/HCl pH 8.0 containing 1 mm EDTA. This preparation is referred to as concentrate. The nearly clear concentrate was filtered using a G3 glass filter and the filter was washed with 80 mL of 20 mm Tris/HCl pH 8.0 containing 1 mm EDTA and 1380 mL clear filtrate was obtained. A fraction of 230 mL was applied on a Sephadex (Pharmancia, Uppsala, Sweden) G75 column (7200 mL column volume, diameter 20 cm, height 23 cm) and eluted with 20 mm Tris, pH 8.0 at 100 mL/min. Fractions containing the target protein (3200-5700 mL) were immediately further processed using anion exchange chromatography. All steps until anion exchange chromatography were performed at 4°C. The enriched fractions of the 12 Sephadex G75 runs were combined, warmed up to 25°C and applied to a 3600 mL Source 15Q (Pharmancia) column (diameter 20 cm, height 12 cm) previously equilibrated with 20 mm Tris, pH 8.0 (loading buffer). After washing with loading buffer, the column was eluted with a 40 L salt gradient of 0-0.25 m NaCl in loading buffer at a flow of 100 mL/min. Fractions of 400 mL were collected and analysed for Ara h 6 content and purity. Purified Ara h 6 was stored in small portions at -20°C. All buffers used were filtered through 0.45 µm Durapore membranes (Millipore, Bedford, MA, USA).

Conglutin is the protein fraction of a peanut kernel comprised of mainly 3 isoforms called Ara h 2 (2 isoforms) and Ara h 6 (1 isoform). Peanut conglutin can be prepared by extracting ground peanut meal, precipitation with ammonium sulphate, and subsequent size exclusion chromatography as described by Koppelman et al., Clin. Exp. Allergy, April 2005, 35(4):490-7.

Protein concentrations in extracts were measured with Bradford analysis (BioRad Laboratories, Hercules, CA, USA) using bovine serum albumin as a standard.

### Peanut modifications

### 1. Glutaraldehyde modification of Ara h1 and Ara h2

Modification with glutaraldehyde was performed by adding a glutaraldehyde to a peanut extract or purified Ara h 1 or Ara h 2 at different pH values (Tables 2-4). After a 4 hours incubation at room temperature, the modified extract was diafiltrated against buffer over a 5 kD membrane. After diafiltration glycine was added to react with residual aldehyde groups. After a second diafiltration against buffer the samples were stored at 2-8°C until analysis.

### 2. Modification of peanut conglutin with DTT, Iodoacetamide and glutaraldehyde

Conglutin was diafiltered and diluted in 5 ml of 100 mM TRIS in absence or presence of 8 M Urea (pH=8.5) at a concentration of 0,5 mg/ml. 0.05 ml of 1M DTT was added and the mixture was incubated for one hour at 56°C. Then, 0.6 ml of 0.5 M Iodoacetamide was added and incubated for 1.5 hours at room temperature. The mixture was diafiltered into 50 mM phosphate buffer (pH=8,0) and the conglutin concentration was readjusted to 0.25 mg/ml in 10 ml. 0.02 ml of a 5% solution of glutaraldehyde was added, and then the mixture was gently shaken overnight at room temperature. An excess of glycin was added to stop the reaction, and the mixture was diafiltered to remove excess of reagents. Instead of 8 M Urea 6M guanidine may be used.

Summarizing, the following samples were prepared:
Untreated conglutin, called native
Reduced and alkylated conglutin, called RA
Reduced and alkylated conglutin prepared in the presence of urea, called RAU
Reduced and alkylated conglutin prepared in the presence of urea, treated afterwards with glutaraldehyde, called RAUGA

### Wasp venom modifications

Wasp venom was diafiltered and diluted in 5 ml of 100 mM TRIS containing 8 M Urea (pH=8.5) at a concentration of 0.5 mg/ml. 0.05 ml of 1M DTT was added and the mixture was incubated for one hour at 56 °C. Then, 0.6 ml of 0.5 M Iodoacetamide was added and incubated for 1.5 hours at room temperature. The mixture was diafiltered into 50 mM phosphate buffer (pH=8.0) and the wasp venom concentration was readjusted to 0.25 mg/ml in 10 ml. 0.02 ml of a 5% solution of glutaraldehyde was added, and then the mixture was gently shaken overnight at room temperature. An excess of glycine was added to stop the reaction, and the mixture was diafiltered to remove excess of reagents. Instead of 8 M Urea 6M guanidine may be used.

### Circular dichroism spectroscopy

CD spectra were recorded on a J-715 CD spectropolarimeter (Jasco) at 25°C. Samples were measured using a 300 µl quartz cuvette (Hellma) with 0.1 cm path length. For far-UV CD measurements (260-195 nm), a protein concentration of 100 µg/ml was used. In case of near-UV CD measurements (350-250 nm), a protein concentration of 500 µg/ml was used. All CD spectra resulted from averaging twenty repeated scans (step resolution 1 nm, scan speed 100 nm/min). Whereas far-UV spectra were only buffer-corrected, near-UV spectra were buffer-corrected and in addition baseline-corrected and smoothed. Far-UV CD spectra were analysed using the program CDNN (CD Spectra Deconvolution, Version 2.1, Böhm, 1997) to predict the secondary structure content of the protein samples.

### Patients

Blood was collected from six peanut-allergic patients, who were included in the study who were previously well-characterized at the department of Dermatology/Allergology (Utrecht, NL). The study was approved by the Ethics Committee of the University Medical Center Utrecht. All patients gave written informed consent. Inclusion criteria were: > 18 years of age, peanut allergy proven by double-blind placebo-controlled food challenge (DBPCFC) or by a clear history, and previously deteremined peanut-specific IgE >3.5 kU/L (preferably >17 kU/L). Previous immunoblot data showed IgE recognition of both Ara h2 and Ara h6 in all patients. Peanut-specific IgE was determined again by CAP upon inclusion in the study. Clinical characteristics of the patients are summarized in Table 1.

**Table 1. Patient characteristics**

| **Patient nr.** | **Code** | **Inclusion IgE peanut** | **Previous IgE peanut** | **Previous Müller^{*}** | **Previous threshold DBPCFC** | **Previous immunoblot Ara h2** | **Previous immunoblot Ara h6** |
|---|---|---|---|---|---|---|---|
| HAL1 | 267979 | >100 | >100 | 4 | 100 mg | > +++ | > +++ |
| HAL2 | 6068671 | 25.6 | 44 | 4 | 0.1 mg | +++ | +++ |
| HAL3 | 2915683 | 47.7 | 85 | 4 | 0.1 mg | > +++ | > +++ |
| HAL4 | 134967 | 78.8 | >100 | 3 | 0.1 mg | > +++ | > +++ |
| HAL5 | 4378052 | 9.25 | 18 | 4 | 10 mg | ++ | ++ |
| HAL6 | 2305667 | 14.8 | 18 | 0 | 10 mg | ++ | ++ |

| | | | | | | | |
|---|---|---|---|---|---|---|---|
| * Müller score (most severe symptoms by history): 0, symptoms of the oral cavity; 3, respiratory symptoms; 4, cardiovascular symptoms. | | | | | | | |

### Immunoblot

IgE recognition of the allergen variants was analyzed by IgE immunoblotting. SDS-PAGE gel electrophoresis and IgE immunoblotting was performed using 15% acrylamide gels. Pre-stained molecular weight markers with molecular weights of 14.3, 21.5, 30, 46, 66, 97.4 and 220 kDa were used as reference. Samples were mixed in a 1 : 1 ratio with 63 mm Tris buffer (pH 6.8) containing 1% dithiotreitol (DTT), 2% SDS, 0.01% bromophenol blue and 20% (v/v) glycerol and were subsequently boiled for 5 min. Gels were loaded with 2 µg CPE, and 1 µg of the purified major peanut allergens Ara h2 and Ara h6, as well as the 4 allergen variants. Gels were stained with Coomassie brilliant blue R-250 dissolved in destaining solution (10% HAc (v/v), 5% methanol (v/v) in water). After destaining, gels were scanned with an ImageMaster DTS (Pharmacia, Uppsala, Sweden). To study the immuno-reactivity of the proteins, SDS-PAGE gels were prepared and the separated proteins were transferred to polyvinyldifluoride sheets (Immobilon-P, Millipore Corp., Bedford, MA, USA). Membranes were blocked with 3% BSA in wash buffer (50 mm Tris, pH 7.5, containing 0.1% BSA and 0.1% Tween 20) for 1 h at room temperature. Patient serum was diluted 50 times, and IgE bound to the membrane was detected with a peroxidase-conjugated goat-anti-human IgE (Kirkegaard and Perry Limited, Gaithersburg, MD, USA).

### Solid-phase IgE-binding test

IgE-binding properties were measured by solid-phase immuno assay (Inhibition ELISA), a method often used for determining the potencies of allergen extracts, for example peanut (Koppelman et al., Biol Chem. 1999; 274(8):4770-7. 1999. Here, a pool of serum obtained from patients with clinical peanut allergy is used. Dilutions of allergen (final protein concentrations: 0.2-30 µg/ml, as was calculated from an allergen batch which protein concentration was determined by the Bradford protein assay (Biorad)) were pre-incubated with a 1:40 dilution of patient serum in phosphate-buffered saline (PBS) containing 0.1% BSA and 0.05% Tween. The allergens were allowed to bind to IgE for 1 h at room temperature. Subsequently, this mixture was loaded on an allergen-coated plate. In this way, the remaining free IgE in the mixture is able to bind to the allergens attached to the plate. IgE bound to the allergen-coated wells was then detected using an anti-human IgE antibody conjugated to horseradish peroxidase. The inhibition of IgE binding as a function of the amount of allergen present in the pre-incubation sample reflects the potency of that allergen variant for IgE. Potencies were compared using the parallel line approach.

### Basophil degranulation

Donor basophils from non-allergic persons: Buffy coats (n=4) were collected at the Blood Bank, National University Hospital of Copenhagen. The Blood Bank has a general ethical approval to hand out buffy coats making sure that the blood donors are anonymous. The buffy coats were initially screened for sensitization against food allergens (n=10) and inhalation allergens (n=10) before included into the study. Only non-allergic, anti-IgE responding buffy coats were used. Donor basophils were semi-purified on lymphopreb (PBMC suspension). Their IgE was removed by a rebounce in pH (down to 3.75 and then back to 7.4) and loaded with IgE from sera from patients described in Table 1 (1 hour sensibilization). The basophils were then stimulated with peanut allergoid or extract (5 dilutions, triplicates) and the released histamine was measured. The presented results (% histamine release) were corrected for background/blank.

### Primary lymphocyte proliferation

Leukocyte stimulation tests (LST) are a model for the first contact of the immune system with (foreign) antigens. An LST contains different (white blood) cell. Upon contact with antigens, APC's will present the antigen to T-cells, which subsequently will proliferate. This assay was performed to check whether the PBMCs that are cultured to generate peanut-specific TCL have a good primary peanut-specific response. Furthermore, the assay provides an impression of the potency of the other allergen extracts to induce primary lymphocyte proliferation. PBMCs were purified from 70 ml venous blood from six peanut allergic patients by Ficoll gradient centrifugation. Cells were cultured (37°C and 5% CO2) in 96-well round-bottom plates in triplicate (2·10⁵ cells/well) in culture medium (IMDM medium containing 5% human serum (HS), penicillin (100 IU/ml), streptomycin (100 mg/ml), and glutamine (1 mmol/ml)) in the presence and absence of CPE, purified Ara h2 and Ara h6, or the 4 allergen variants (all at 50 µg/ml). After 6 days of culture, supernatants were taken for measurements of cytokines (IL-10, IL-13, IFN-γ, TNF-α). For proliferation, [3H]-TdR (0.75 µCi/well) was added at day 6 for overnight incubation, cells were harvested and incorporation of [3H]-TdR was measured using a 1205 β-plate counter (Wallac, Turku, Finland) and expressed as counts per minute (cpm). Proliferation is expressed as stimulation index (SI; proliferation to allergen stimulation divided by blank). It is desired that the SI of the modified protein is close to or higher than the SI of the unmodified protein. An SI>2 is considered positive. PBMCs that were left were stored in liquid nitrogen.

### Peanut-specific T cell lines (TCLs)

To be even more specific, (short) T-cell lines can be prepared by culturing with isolated allergens such as Ara h 2 and Ara h 6. Proliferation is considered to be a measure for immunogenicity required for effective immunotherapy. PBMCs were cultured in 48-well flat-bottom plates in triplicate (10⁶ cells/well) in culture medium in the presence of CPE (50 µg/ml), or a mixture of purified Ara h2 and Ara h6 (both 50 µg/ml). IL-2 was added to the cultures (10 U/ml) at day 7. At day 11, TCLs were restimulated in two wells in a 24-well flat-bottom plate with feedermix containing irradiated allogenic PBMCs (2 donors, 5_{'}10⁵ cells/well) and EBV-transformed B-cells (1x10⁵ cells/well), IL-2 (10 IU/ml), and PHA as mitogen (0.5 µg/ml). At day 21, TCLs were tested for antigen-specificity in 96-well round bottom plates (3·10⁴ cells/well) by stimulation with autologous PBMCs (1·10⁵ cells/well) in the absence or presence of CPE, Ara h2, Ara h6, and the 4 allergen variants (all at 100, 50 and 25 µg/ml). After 48 hours, supernatants were taken for cytokine measurements and 0.75 µCi/well of [3H]-TdR was added for overnight incubation. Cells were harvested and incorporation of [3H]-TdR was measured using a 1205 β-plate counter (Wallac, Turku, Finland) and expressed as counts per minute (cpm). The stimulation index was the measured counts per minute in the presence of antigen divided by the measured counts per minute in the absence of antigen.

### RESULTS

### GA modification of peanut allergens

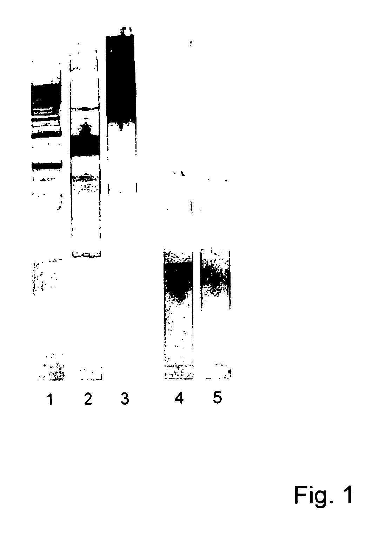

Modification of peanut allergens with glutaraldehyde reduces the IgE-binding to some extent, but not sufficiently, even though optimization (pH, concentration of reagents, incubation temperature and time, Tables 1, 2, and 3) has been applied to the reaction conditions. To further investigate the effect of gluteraldehyde treatment on IgE-binding, SDS-PAGE was performed in combination with IgE-blotting using 12.5% gel precast gels (Amersham Biosciences) and PVDF membranes. Figure 1 shows the results of purified Ara h 1 and purified Ara h 2. Modification of Ara h 1 results in loss of almost all the individual bands (Figure 1, lane 3) but not the loss of IgE-binding activity, as the intensity of the bands in lane three is not less than that in lane 2. For Ara h 2, no change in molecular weight is observed. Next to the observation that the molecular weight of Ara h 2 is not increased significantly upon treatment with glutaraldehyde, the IgE binding of the glutaraldehyde-treated allergens on blot is not decreased. This indicates that modification by gluteraldehyde on the molecular weight of Ara h 2 and IgE-binding is limited.

**Table 2. Modification results of a whole peanut extract at different pH's and different concentrations of glutaraldehyde**

| **pH** | **Glutaraldehyde 50% µl per mg protein** | **Residual activity** |
|---|---|---|
| 7.2 | 0.6 | 28 |
| 7.2 | 1.2 | 22 |
| 8.0 | 0.6 | 22 |
| 8.0 | 1.2 | 27 |
| 8.0 | 1.8 | 28 |
| 9.0 | 0.6 | 33 |
| 9.0 | 1.2 | 28 |
| 9.0 | 1.8 | 25 |

**Table 3. Ara h1 modified with glutaraldehyde**

| **pH** | **Glutaraldehyde 50% µl per mg protein** | **Residual activity %** |
|---|---|---|
| 7.2 | 1.2 | 40 |
| 7.2 | 1.8 | 44 |

**Table 4. Ara h2 modified with glutaraldehyde**

| **pH** | **Glutaraldehyde 50% µl per mg protein** | **Residual activity %** |
|---|---|---|
| 7.2 | 1.2 | 52 |
| 7.2 | 1.8 | 37 |

### Combined RA and GA modification of peanut allergens

As expected, RA treatment resulted in a loss of Cys residues as determined with standard amino acid analysis (Table 5). GA treatment after RA treatment resulted in a loss of Lys residues as determined with standard amino acid analysis, which is unexpected because GA treatment alone (Figure 1, Tables 1-3) did not result in sufficient modification of the Ara h 2 on a functional level (IgE-binding):

**Table 5. Degree of modification after RA and GA treatment**

| | Modified amino acids (%) | |
|---|---|---|
| | Cys | Lys |
| 1.Untreated | 0 | 0 |
| 2.RA | 99 | 0 |
| 3.RAU | 69 | 0 |
| 4.RAGA | 85 | 55 |
| 5.RAUGA | 88 | 59 |

### Characterization of peanut protein structure after RA and GA modification

### Secondary protein folding level

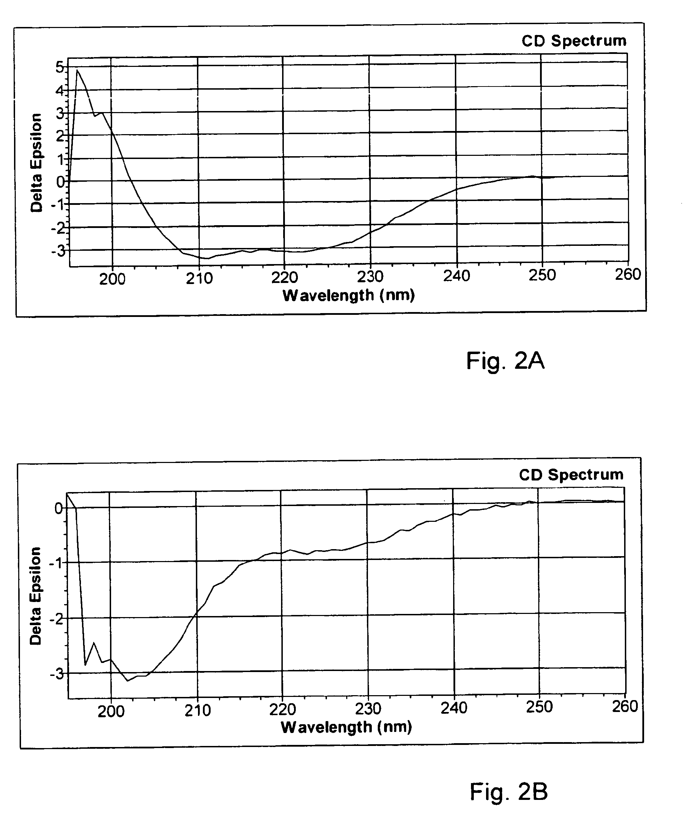

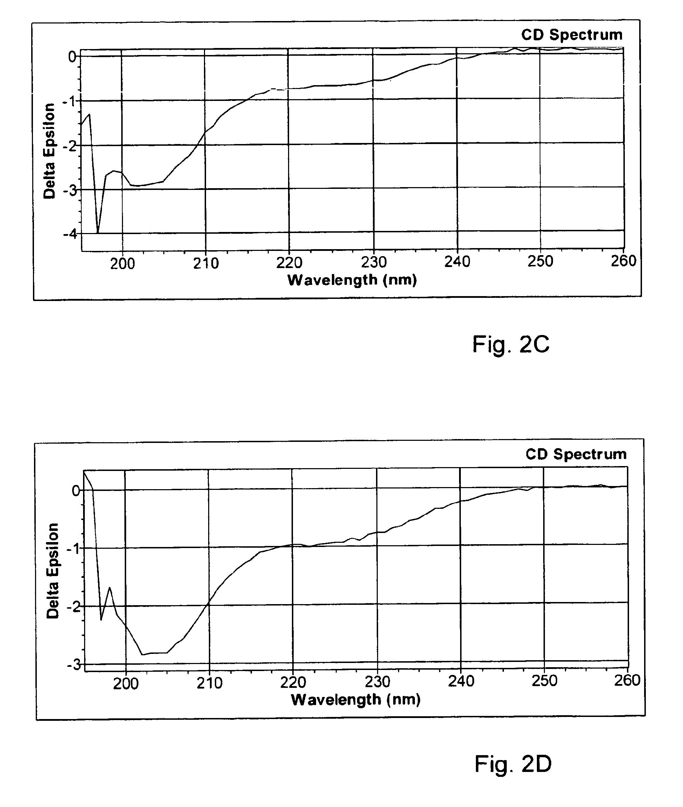

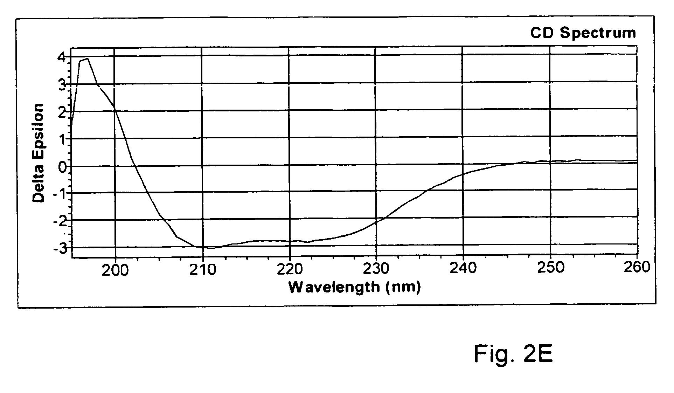

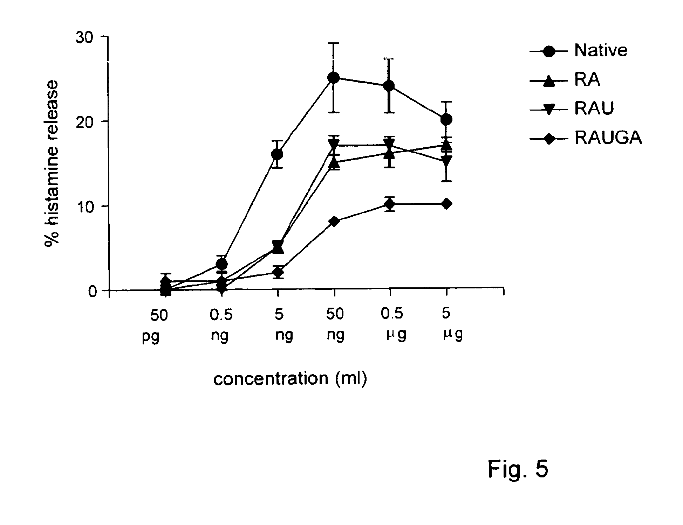

The consequences of modification and double modification were investigated on the level of secondary protein structure. Far UV circular dichroism spectra were recorded (195-260 nm) and the percentages of secondary structure elements were calculated. Figure 2 shows the individual far UV CD spectra and Table 6 summarizes the corresponding secondary structure elements. From Figure 5 it is clear that RA modification in presence or absence of urea, and with or without GA treatment results in a dramatic change of the spectrum. The fact that the ellipticity around 220 nm has decreased is indicative for loss of helical structures and formation of random coil (denaturation of protein). Furthermore, the minimum has shifted to approx. 205 nm which is indicative for the formation of beta-structure. These findings were supported by the calculations on the secondary structure (Table 6).

**Table 6. Secondary structure elements of conglutins after RA and/or GA treatment**

| | **Reference** | **RA** | **RAU** | **RAUGA** | **GA** |
|---|---|---|---|---|---|
| **Helix** | 34.30% | 17.30% | 16.70% | 17.90% | 31.60% |
| **Antiparallel** | 8.10% | 16.90% | 17.40% | 16.40% | 8.90% |
| **Parallel** | 8.70% | 14.20% | 14.70% | 14.00% | 9.40% |
| **Beta-turn** | 16.60% | 21.20% | 21.40% | 21.00% | 17.10% |
| **Random coil** | 32.70% | 42.40% | 43.40% | 42.30% | 34.60% |
| **Total sum** | 100.30% | 112.10% | 113.60% | 111.50% | 101.50% |

While the modification of peanut conglutin with only GA does not result in a change of secondary structure, RA treatment reduces the helical content resulting in an increase of random coil and beta-structure. These protein transformations have been observed earlier for Ber e 1, a conglutin-like protein from Brazil nut [Koppelman et al., J. Agric. Food Chem., 2005, 53(1), pp. 123-31].

### Tertiary protein folding level

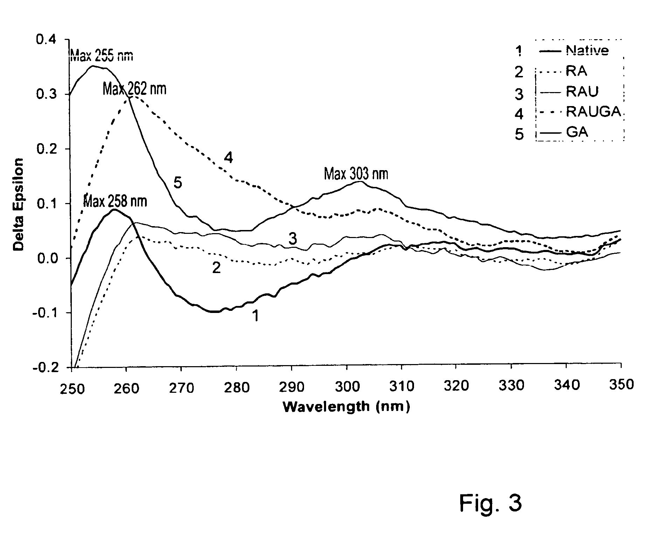

The consequences of modification and double modification were also investigated on the level of tertiary protein structure by near UV circular dichroism (250-350 nm). Figure 3 shows the near UV CD spectra of native and modified conglutin. The fact that RA and RAU modified conglutin spectra (dashed lines) show hardly any ellipticity confirms denaturation of the protein (formation of random coil). Native, GA treated and RAUGA treated protein all show ellipticity with absorption maxima at 258, 255 and 262 nm, respectively. These maxima are indicative for phenylalanine (250-270 nm) and alterations in its environment. Conglutin contains three phenylalanins and one of them is located in a helix next to a lysine. The binding of GA to this lysine in case conglutin is treated with GA appears to change the environment of phenylalanine resulting in a shift of the absorption maximum (from 258 to 255 nm). As described above, spectra of RA and RAU modified conglutin did not show any signal. The use of GA after reduction-alkylation seems to regain asymmetry in the area of phenylalanine as an absorption maximum at 262 nm was observed in the RAUGA spectrum. This maximum differs from the maxima observed in the spectra of native and GA-treated conglutin which means that the environment of the phenylalanine in these three samples differs. No signals of tyrosine (270-290 nm) and tryptophan (280-300 nm) in all spectra can be explained by the fact that these residues are located in random coils of the protein.

### IgE binding properties of modified peanut conglutin

### Solid-phase IgE-binding test

IgE-binding properties were measured by solid-phase immuno assay using a pool of serum obtained from patients with clinical peanut allergy. A sample with unchanged IgE-binding properties would have a potency of 100%. A sample in which no IgE-binding is left would measure 0%. Table 7 shows the potencies for differently treated samples. The relative potency of the modified product is in all 3 cases lower than 1%. However, the RAUGA variant shows an even lower IgE-binding property compared to RA and RAU.

**Table 7. IgE-binding potencies of conglutins after RA and GA treatment**

| **Sample** | **Potency** | |
|---|---|---|
| Native | 100% | |
| RA | 0.6% | |
| RAU | 0.6% | |
| RAUGA | 0.1% | |

### SDS-PAGE and IgE-immunoblotting

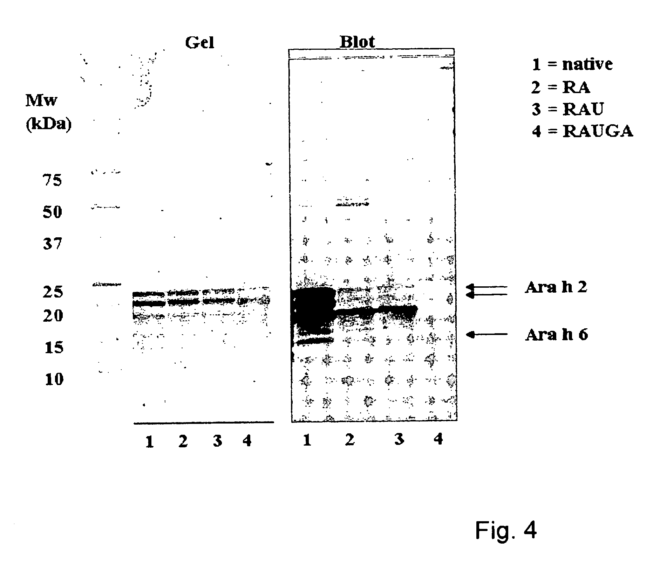

The molecular weight of conglutin is not affected substantially upon RA treatment. The presence of urea or modification with GA does not change the molecular weight (Figure 4). To further substantiate the IgE-binding properties, IgE-immunoblotting combined with SDS-PAGE was performed. RA and RAU already have low IgE-binding (Figure 4, right panel, lane 2 and 3), and RAUGA reduced IgE-binding even stronger (Figure 4, right panel, lane 4) and no immune response could be detected on the blot. IgE-blots were repeated with individual patient sera, and IgE binding was scored semi-quantitatively (Table 8). Residual IgE binding to RA and RAU were 30 to 70%, while for RAUGA 0 - 10%, illustrating the added value of the double modification.

**Table 8. IgE-binding potencies of conglutins after RA and GA treatment, individual patient sera used in (semi-quantitative) IgE-blot**

| Patient | Ara h2 | | | | Arah6 | | | |
|---|---|---|---|---|---|---|---|---|
| | native | RA | RAU | RAUGA | native | RA | RAU | RAUGA |
| HAL1 | 100% (5+) | 70% | 60% | 10% | 100% (5+) | 70% | 60% | 10% |
| HAL2 | 100% (3+) | 60% | 30% | 0% | 100% (3+) | 10% | 5% | 0% |
| HAL3 | 100% (3+) | 60% | 60% | 10% | 100% (3+) | 10% | 10% | 0% |
| HAL4 | 100% (4+) | 70% | 50% | 10% | 100% (4+) | 70% | 40% | 0% |
| HAL5 | 100% (2+) | 60% | 60% | 5% | 100% (2+) | 40% | 40% | 0% |
| HAL6 | 100% (3+) | 60% | 40% | 10% | 100% (3+) | 30% | 10% | 0% |
| Mean | 100% (3.3+) | 63% | 50% | 8% | 100% (3.3+) | 38% | 28% | 2% |

### Basophil histamine release by modified peanut allergens

The potency of the 6 different sera (strength in sensitizing basophils) to the 4 allergen variants was tested. RA, RAU and RAUGA are poorer in inducing a histamine release from donor basophils sensitized with serum from peanut allergic patients. In Figure 5, an example of histamine release for one of the patients is shown. Native conglutin induces histamine release (HR) already at low concentrations. RA and RAU show a similar decreased ability to induce HR. Unexpectedly RAUGA induces even less HR, as observed by a later onset of HR and a lower plateau. Concentrations required for 10% HR, which is considered a relevant threshold for histamine release, are for RA-treated conglutin between 5 and 50 ng/ml, and for RA-treated conglutin treated with GA afterwards between 500 and 5000 ng/ml, a hundred fold higher, indicating a hundred fold decrease in potency.

### T cell proliferation

Data are given for three types of peanut allergic patients: With low peanut specific IgE, with moderate, and with high peanut specific IgE.

### Leukocyte stimulation test (LST)

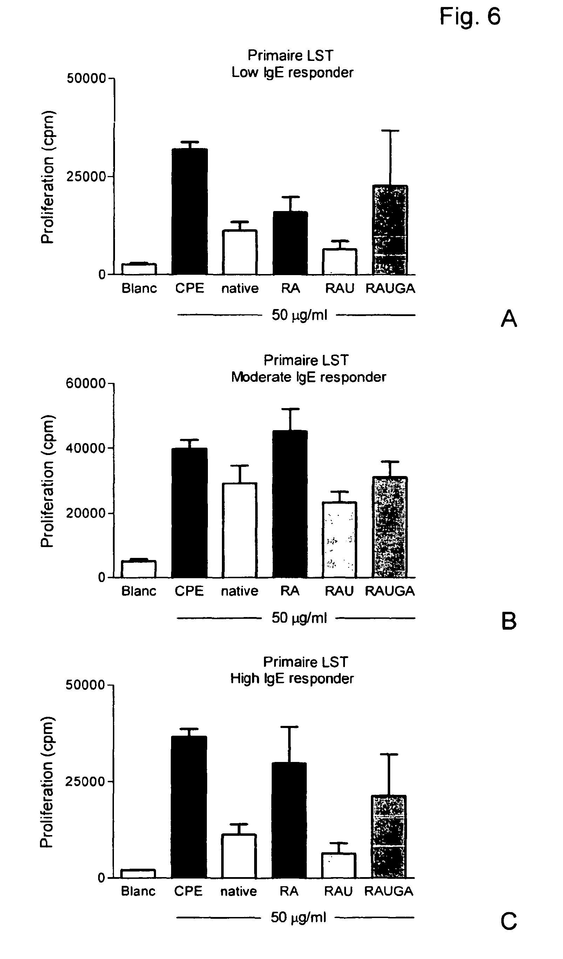

All donors (6/6) had a strong proliferation upon CPE stimulation. Data for 3 different patients (With low peanut specific IgE, with moderate, and with high peanut specific IgE) are shown in Figure 6. The response to Ara h2 was weaker in stimulation index (SI) than the response to Ara h6. It is noted that RA treatment results in a higher proliferation than native, the response to RAUGA was comparable and the response to RAU was lower as compared to native extract. Considering that the same proteins are present, and in the same concentration, another factor must cause the enhanced proliferation. Probably, the increased digestibility of conglutins after reduction and alkylation explains this. Increased digestibility may turn conglutins into better substrates for antigen-presenting cells. In contrast to what has often been described for GA treatment of allergens, in this case, after RA treatment, treatment with GA does not result in decreased proliferation.

### CPE-specific T-cells

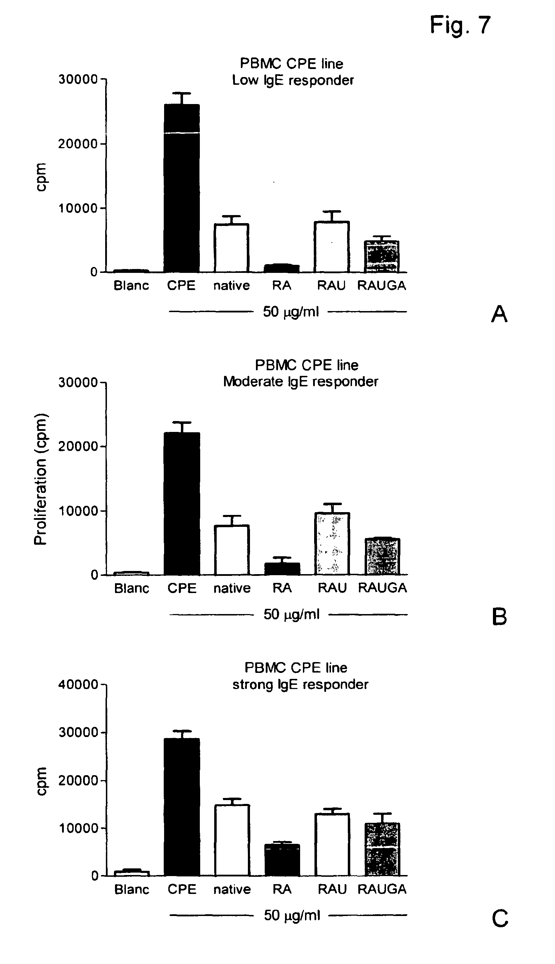

The analysis of the T cell response to a specific allergen is more specific and more optimal after pre-selection of allergen-specifis T cells by the generation of short-term TCLs. Therefore, short-term TCLs were generated specific for CPE. Data for 3 different patients (With low peanut specific IgE, with moderate, and with high peanut specific IgE) are shown in Figure 7. It is noted that RA treatment results in a lower proliferation than native. Surprisingly, treatment with GA after RA restores the proliferative responses of RA treated sample. This effect is most pronounced for the patient with the lowest peanut-specific IgE. In contrast to what has often been described for GA treatment of allergens, in this case, after RA treatment, treatment with GA does not result in decreased proliferation, but in an improved proliferation. It is also interesting to note the RA treatment in the presence of Urea (RAU) results in a higher T-cell proliferation in this model system.

### Ara h 2/Ara h 6-specific T-cells

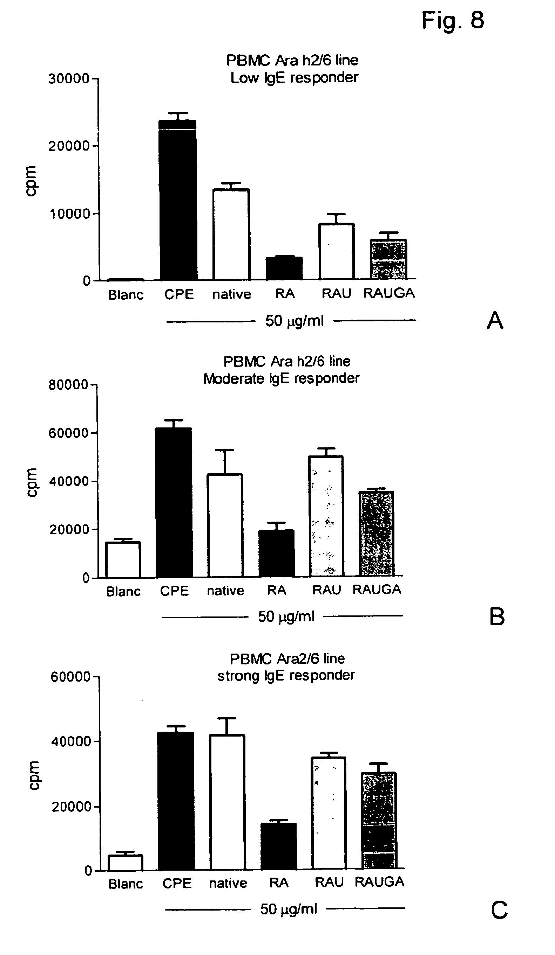

Data for 3 different patients (With low peanut specific IgE, with moderate, and with high peanut specific IgE) are shown in Figure 8. The data support the observations of the previous mentioned T-cell data obtained with T-cells for peanut extract. All TCLs had a very low background proliferation, with high proliferation upon CPE stimulation. This shows that, indeed, the generation of peanut-specific TCLs enhanced the peanut-specific response (mean SI to CPE around 50) as compared to the primary response (mean SI to CPE around 12). None of the TCLs responded to parvalbumin, which was included to check the TCLs for peanut-specificity.

### Modification of wasp venom by RA and GA treatment

The IgE-binding potency was determined as for conglutin, using in the present case a pool of sera from wasp venom-allergic patients. Table 9 shows the results.

**Table 9. Potency of wasp venom sample treated with RA and GA**

| **Sample** | **Modification applied** | **Potency** |
|---|---|---|
| Native | None | 100% |
| RA | Reduced and alkylated | 14% |
| RAUGA | Reduced and alkylated, and glutaraldehyde treated | 0% |
| GA | glutaraldehyde treated | n.a. due to protein precipitation |

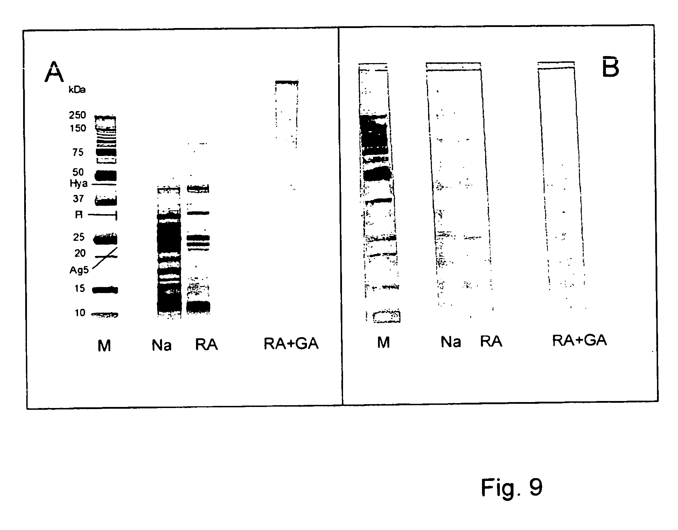

Figure 9 shows the SDS-PAGE pattern and IgE blot of the native and treated wasp venom. It is clear from Table 9 that gluteraldehyde treatment alone, as is common for other allergens, is not suitable for wasp venom under the chosen conditions because of precipitation of the wasp venom. Reduction and alkylation reduces the IgE binding substantially to 14%. Surprisingly, subsequent treatment with gluteraldehyde further decreases the IgE binding without excessive precipitation of the wasp venom.

### CONCLUSIONS

Double modification peanut conglutin by RA and GA treatment has been performed for peanut and wasp venom allergens. While the modification of peanut conglutin with GA only does not result in a change of secondary structure, RA treatment reduces the helical content resulting in an increase of random coil and beta-structures. Furthermore, RA treatment followed by GA modification results in a tertiary structure that differs from that of conglutin treated only with RA. It appears that in case of the double modification not only the Cys residues are modified, but also the Lys residues.

Double modification of peanut conglutin by RA and GA treatment leads to a pronounced reduction of IgE binding, also in functional way (basophil histamine release). The additional effect of GA compared to RA alone is surprising because GA treatment alone did not result in substantial decrease of potency. RA conglutin has decreased IgE-binding as compared to native, demonstrated by IgE-ELISA, IgE blot, and BHR. Treatment with GA after RA pronounces this effect up to a hundred fold. This is unexpected because GA treatment without pre-treatment by RA does not decrease IgE binding substantially (only 2-3 fold, Figure 1). Our data show that all 3 tested modifications lead to a reduction in IgE binding, with the strongest reduction observed after both reduction/alkylation and glutaraldehyde treatment (RAUGA).

The double modification of wasp venom also results in a strongly diminished IgE-binding, far more pronounced that RA treatment alone. This was surprising because GA treatment without preceding RA treatments was not successful due to precipitation.

T cell proliferation tests were performed where PBMC responses can be affected by the presence of multiple cell types and therefore the clearest conclusions can be drawn from the data obtained with the antigen-specific TCLs. For immunotherapy, the best option would be a modification which leads to (near-) complete reduction of IgE-binding, and maintenance of T cell responses which is needed for immunomodulation. From the 3 modified peanut proteins, RAU induced a good T cell response whereas IgE binding was reduced substantially as described above. The IgE binding to RA was slightly less reduced than to RAU, and the T cell response was less strong, which suggest that this modification is less optimal for application in SIT. IgE-binding to RAUGA was reduced almost completely and RAUGA also induced a strong T cell response. In that respect, RAUGA would be the best candidate. For venom allergens, the effect of the double modification with RA and GA on IgE-binding has been evaluated. While GA treatment alone results in protein precipitation, pretreatment with RA leads to an almost complete reduction of IgE binding, while the proteins remained soluble.

### LEGENDS OF THE FIGURES

**Figure 1****:** IgE-blot of glutaraldehyde treatment purified Ara h 1 and Ara h 2, showing marker proteins (lane 1), unmodified Ara h 1 (lane 2), modified Ara h 1 (lane 3), unmodified Ara h 2 (lane 4), modified Ara h 2 (lane 5).
**Figure 2****:** Far UV CD spectra of conglutin before and after modifications with **A;** Native, untreated conglutin, **B;** RA treated conglutin, **C;** RAU treated conglutin, **D;** RAUGA treated conglutin and **E;** GA treated conglutin. CD spectra were recorded on a J-715 CD spectropolarimeter (Jasco) at 25°C. Samples were measured using a 300 µl quartz cuvette (Hellma) with 0.1 cm path length and a protein concentration of 100 µg/ml was used. CD spectra resulted from averaging twenty repeated scans (step resolution 1 nm, scan speed 100 nm/min) and were buffer-corrected afterwards.
**Figure 3****:** Near UV CD spectra of conglutin before and after modifications. Near UV CD spectra of native conglutin (black line), RA treated conglutin (dashed grey line), RAU treated conglutin (dashed black line), RAUGA treated conglutin (dark grey line) and GA treated conglutin (grey line). CD spectra were recorded on a J-715 CD spectropolarimeter (Jasco) at 25°C. Samples were measured using a 300 µl quartz cuvette (Hellma) with 0.1 cm path length, a protein concentration of 500 µg/ml was used. CD spectra resulted from averaging twenty repeated scans (step resolution 1 nm, scan speed 100 nm/min) and were buffer-corrected afterwards and in addition baseline-corrected and smoothed.
**Figure 4****:** Double modification of peanut conglutinin (RA + GA). SDS-PAGE pattern (left panel) and IgE blot (right panel) of the native and treated wasp venom. Marker proteins (Mw) are indicated on the left, both the gel and the blot contain lanes with native conglutinin (1), reduced and alkylated conglutinin (2), reduced, alkylated conglutinin with the addition of urea (3) and reduced, alkylated conglutinin with urea and treated with glutaraldehyde (4).
**Figure 5****:** Graphic illustration of an example of the percentage histamine release from a peanut-allergic patient. Basophils are stripped from IgE, re-loaded with IgE from the allergic patient and stimulated with peanut allergoid or extract (triplicates). The presented results (% histamine release) are corrected for background/blank.
**Figure 6****:** Primary LST responses to crude peanut extract (CPE), native (combination Ara h2 and Ara h6) and modified Ara h2/Ara h6 (RA, RAU, RAUGA). Triplicate cultures of a mild (**A**), moderate (**B**) and highly peanut-allergic patient (**C**) were stimulated with 50 µg/ml of allergen or allergoid. Cells cultured in medium were served as control. Six days later, the cultures received an 18-hour pulse of 1 uci per well of thymidine. Cells were harvested, and the incorporated radioactivity was counted the results are expressed as counts per minute.
**Figure 7****:** Fresh crude peanut extract (CPE)-specific PBMC responses to CPE, native (combination Ara h2 and Ara h6) and modified Ara h2/Ara h6 (RA, RAU, RAUGA). Triplicate cultures of a mild (**A**), moderate (**B**) and highly peanut-allergic patient (**C**) were stimulated with 50 µg/ml of allergen or allergoid. Cells cultured in medium were served as control. Six days later, the cultures received an 18-hour pulse of 1 uci per well of thymidine. Cells were harvested, and the incorporated radioactivity was counted the results are expressed as counts per minute.
**Figure 8****:** Fresh Ara h2/Ara h6-specific PBMC responses to crude peanut extract (CPE), native (combination Ara h2 and Ara h6) and modified Ara h2/Ara h6 (RA, RAU, RAUGA). Triplicate cultures of a mild (A), moderate (B) and highly peanut-allergic patient (C) were stimulated with 50 µg/ml of allergen or allergoid. Cells cultured in medium were served as control. Six days later, the cultures received an 18-hour pulse of 1 uci per well of thymidine. Cells were harvested, and the incorporated radioactivity was counted the results are expressed as counts per minute.
**Figure 9****:** Double modification of wasp venom (RA + GA). SDS-PAGE pattern (panel A) and IgE blot (panel B) of the native and treated wasp venom. Marker proteins (M) are indicated on the left, both the gel and the blot contain lanes with native wasp venom (Na), reduced and alkylated wasp venom (RA) and reduced, alkylated and glutaraldehyde treated wasp venom (RA + GA).

## Claims

1. A process for modifying an allergen comprising the steps of reduction, alkylation and treatment with a cross-linking agent, wherein the allergen is a protein comprising cystein residues, wherein reduction is carried out prior to alkylation and wherein the treatment with the cross-linking agent is carried out after reduction and alkylation.

2. A process according to claim 1, wherein the cross-linking agent is an aldehyde.

3. A process according to claim 2, wherein the cross-linking agent is glutaraldehyde.

4. A process according to any of the claims 1 to 3, wherein the reduction is carried out using a reducing agent chosen from the group of 2-mercaptoethanol (β-ME), dithiothreitol (DTT), dithioerythritol, cysteine, homocystein, tributylphosphine, sulfite, tris(2-carboxyethyl) phosphine (TCEP), sodium (cyano) borohydride, lye, glutathione, E-mercapto ethylamine, thioglycollic acid, methyl sulfide, ethyl sulfide and combinations thereof.

5. A process according to any of the preceding claims, wherein the alkylation is carried out using an alkylating agent chosen from the group of N-ethylmalimide, cystamine, iodoacetamide, iodoacetic acid, alkylhalogenides, alkylsulfates, alkenes, terminal alkenes (H₂C)=C(H)-R, enzymes and combinations thereof.

6. A process according to any of the preceding claims, wherein the allergen is a recombinant protein or a synthetic peptide.

7. A process according to any of the preceding claims, wherein the allergen is obtained from a vegetable source, from a vegetable storage protein, from an insect, from insect venom, from a mammal or a fish or crustacean, from an expression system for recombinant proteins, from a bacterium expression system for recombinant proteins, from a yeast expression system for recombinant proteins or from an microorganism expression system.

8. An allergen obtainable by a process according to any of the preceding claims.

9. Allergen according to claim 8 for use as a medicament.

10. Allergen according to claim 8 for use in immunotherapy treatment of an allergy brought about by the native form of, or cross-reactive with said allergenic protein.

11. A pharmaceutical composition comprising an allergen according to claim 8 and a pharmaceutically acceptable carrier and/or an adjuvant.

12. A pharmaceutical composition according to claim 11 having the form of a dosage form chosen from the group of a capsule, tablet, lozenge, dragee, pill, droplets, suppository, aerosol, powder, spray, vaccine, ointment, paste, cream, inhalant, or patch.

13. A pharmaceutical composition according to claim 11 or 12 for use as a medicament.

14. A pharmaceutical composition according to claim 11 or 12 for use in immunotherapy treatment.

15. A pharmaceutical composition according to claim 11 or 12 for use in immunotherapy treatment comprising administering said composition to an allergic patient in need thereof in a pharmaceutically effective dose.

## Patentansprüche

1. Verfahren zur Modifizierung eines Allergens, umfassend die Schritte Reduktion, Alkylierung und Behandlung mit einem Vernetzungsmittel, wobei das Allergen ein Protein ist, das Cystein-Reste umfasst, wobei die Reduktion vor der Alkylierung ausgeführt wird, und wobei die Behandlung mit dem Vernetzungsmittel nach der Reduktion und Alkylierung ausgeführt wird.

2. Verfahren gemäß Anspruch 1, wobei das Vernetzungsmittel ein Aldehyd ist.

3. Verfahren gemäß Anspruch 2, wobei das Vernetzungsmittel Glutaraldehyd ist.

4. Verfahren gemäß irgendeinem der Ansprüche 1 bis 3, wobei die Reduktion unter Verwendung eines reduzierenden Agens, ausgewählt aus der Gruppe aus 2-Mercaptoethanol (β-ME), Dithiothreitol (DTT), Dithioerythritol, Cystein, Homocystein, Tributylphosphin, Sulfit, Tris(2-carboxyethyl)phosphin (TCEP), Natrium (Cyano) Borhydrid, Lauge, Glutathion, E-Mercaptoethylamin, Thioglykolsäure, Methylsulfid, Ethylsulfid, und Kombinationen davon, ausgeführt wird.

5. Verfahren gemäß irgendeinem der vorhergehenden Ansprüche, wobei die Alkylierung unter Verwendung eines Alkylierungsmittels, ausgewählt aus der Gruppe N-Ethylmalimid, Cystamin, Iodacetamid, Iodessigsäure, Alkylhalogenide, Alkylsulfate, Alkene, terminale Alkene (H₂C)=C(H)-R, Enzyme, und Kombinationen davon, ausgeführt wird.

6. Verfahren gemäß irgendeinem der vorhergehenden Ansprüche, wobei das Allergen ein rekombinantes Protein oder ein synthetisches Peptid ist.

7. Verfahren gemäß irgendeinem der vorhergehenden Ansprüche, wobei das Allergen erhalten ist aus einer pflanzlichen Quelle, aus einem pflanzlichen Speicherprotein, aus einem Insekt, aus Insektengift, aus einem Säuger oder einem Fisch oder Krustentier, aus einem Expressionssystem für rekombinante Proteine, aus einem Bakterien-Expressionssystem für rekombinante Proteine, aus einem Hefe-Expressionssystem für rekombinante Proteine, oder aus einem Mikroorganismus-Expressionssystem.

8. Allergen, erhalten durch ein Verfahren gemäß irgendeinem der vorhergehenden Ansprüche.

9. Allergen gemäß Anspruch 8 zur Verwendung als ein Medikament.

10. Allergen gemäß Anspruch 8 zur Verwendung in einer Immuntherapiebehandlung einer Allergie, die durch die native Form von, oder durch Kreuz-Reaktivität mit, diesem allergenen Protein hervorgerufen wurde.

11. Pharmazeutische Zusammensetzung, umfassend ein Allergen gemäß Anspruch 8 und einen pharmazeutisch akzeptablen Träger und/oder einen Hilfsstoff.

12. Pharmazeutische Zusammensetzung gemäß Anspruch 11, welche die Form einer Darreichungsform hat, welche ausgewählt ist aus der Gruppe aus einer Kapsel, einer Tablette, einer Pastille, eines Dragees, einer Pille, Tropfen, Zäpfchen, eines Aerosols, eines Puders, eines Sprays, eines Vakzins, einer Salbe, einer Paste, einer Creme, eines Inhalationsmittels oder eines Pflasters.

13. Pharmazeutische Zusammensetzung gemäß Anspruch 11 oder 12 zur Verwendung als ein Medikament.

14. Pharmazeutische Zusammensetzung gemäß Anspruch 11 oder 12 zur Verwendung in Immuntherapiebehandlung.

15. Pharmazeutische Zusammensetzung gemäß Anspruch 11 oder 12 zur Verwendung in Immuntherapiebehandlung, umfassend Verabreichen dieser Zusammensetzung an einen allergischen Patienten, der ein Bedürfnis danach hat, in einer pharmazeutisch wirksamen Dosis.

## Revendications

1. Procédé permettant de modifier un allergène comprenant les étapes de réduction, d'alkylation et de traitement avec un agent de réticulation, où l'allergène est une protéine comprenant des résidus de cystéine, où la réduction est réalisée avant l'alkylation et où le traitement avec l'agent de réticulation est réalisé après la réduction et l'alkylation.

2. Procédé selon la revendication 1, dans lequel l'agent de réticulation est un aldéhyde.

3. Procédé selon la revendication 2, dans lequel l'agent de réticulation est le glutaraldéhyde.

4. Procédé selon l'une quelconque des revendications 1 à 3, dans lequel la réduction est réalisée en utilisant un agent réducteur choisi dans le groupe du 2-mercaptoéthanol β-ME), du dithiothréitol (DTT), du dithioérythritol, de la cystéine, de l'homocystéine, de la tributylphosphine, du sulfite, de la tris(2-carboxyéthyl)phosphine (TCEP), du (cyano)borohydrure de sodium, de la soude caustique, du glutathion, de l'E-mercapto-éthylamine, de l'acide thioglycolique, du sulfure de méthyle, du sulfure d'éthyle et de combinaisons de ceux-ci.

5. Procédé selon l'une quelconque des revendications précédentes, dans lequel l'alkylation est réalisée en utilisant un agent alkylant choisi dans le groupe du N-éthylmalimide, de la cystamine, de l'iodoacétamide, de l'acide iodoacétique, d'alkyl-halogénures, d'alkylsulfates, d'alcènes, d'alcènes terminaux (H₂C)=C(H)-R, d'enzymes et de combinaisons de ceux-ci.

6. Procédé selon l'une quelconque des revendications précédentes, dans lequel l'allergène est une protéine recombinante ou un peptide de synthèse.

7. Procédé selon l'une quelconque des revendications précédentes, dans lequel l'allergène est obtenu à partir d'une source végétale, d'une protéine de stockage végétale, d'un insecte, d'un venin d'insecte, d'un mammifère ou d'un poisson ou d'un crustacé, d'un système d'expression pour des protéines recombinantes, d'un système d'expression bactérien pour des protéines recombinantes, d'un système d'expression de levure pour des protéines recombinantes ou d'un système d'expression de micro-organisme.

8. Allergène pouvant être obtenu par un procédé selon l'une quelconque des revendications précédentes.

9. Allergène selon la revendication 8 pour une utilisation en tant que médicament.

10. Allergène selon la revendication 8 pour une utilisation dans un traitement d'immunothérapie d'une allergie provoquée par la forme native de, ou présentant une réaction croisée avec, ladite protéine allergénique.

11. Composition pharmaceutique comprenant un allergène selon la revendication 8 et un support et/ou un adjuvant pharmaceutiquement acceptable.

12. Composition pharmaceutique selon la revendication 11 ayant la forme d'une forme galénique choisie dans le groupe d'une capsule, d'un comprimé, d'une pastille, d'une dragée, d'une pilule, de gouttelettes, d'un suppositoire, d'un aérosol, d'une poudre, d'un spray, d'un vaccin, d'une pommade, d'une pâte, d'une crème, d'un produit à inhaler ou d'un patch.

13. Composition pharmaceutique selon la revendication 11 ou 12 pour une utilisation en tant que médicament.

14. Composition pharmaceutique selon la revendication 11 ou 12 pour une utilisation dans un traitement d'immunothérapie.

15. Composition pharmaceutique selon la revendication 11 ou 12 pour une utilisation dans un traitement d'immunothérapie comprenant l'administration de ladite composition à un patient allergique ayant besoin de celle-ci dans une dose pharmaceutiquement efficace.
